(19) Europäisches Patentamt
European Patent Office
Office européen des brevets

(11) EP 4 523 708 A1

(12) EUROPEAN PATENT APPLICATION
published in accordance with Art. 153(4) EPC

(43) Date of publication:
**19.03.2025 Bulletin 2025/12**

(21) Application number: **23823491.8**

(22) Date of filing: **31.03.2023**

(51) International Patent Classification (IPC):
***A61K 48/00*** (2006.01) ***A61P 31/12*** (2006.01)
***A61K 9/10*** (2006.01) ***A61K 9/51*** (2006.01)
***A61K 47/24*** (2006.01) ***A61K 47/36*** (2006.01)
***A61K 31/7088*** (2006.01)

(52) Cooperative Patent Classification (CPC):
**A61K 9/10; A61K 9/51; A61K 31/7088; A61K 47/24; A61K 47/36; A61K 48/00; A61P 31/12**

(86) International application number:
**PCT/JP2023/013650**

(87) International publication number:
**WO 2023/243187 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.06.2022 JP 2022095841**

(71) Applicant: **Kewpie Corporation**
**Tokyo 150-0002 (JP)**

(72) Inventor: **AMANO, Yohei**
**Chofu-shi, Tokyo 182-0002 (JP)**

(74) Representative: **Winter, Brandl - Partnerschaft mbB**
**Alois-Steinecker-Straße 22**
**85354 Freising (DE)**

# (54) LIPID NANOPARTICLES AND PRODUCTION METHOD FOR SAME

(57) The present invention relates to a production method for a lipid nanoparticle, the method including: a step of dissolving a hyaluronic acid derivative in an aqueous solvent to form an aqueous phase; a step of dissolving a phospholipid in an organic solvent to form an oil phase; and a step of dropping and dispersing the oil phase into the aqueous phase, wherein the hyaluronic acid derivative is represented by a following general formula (1):

[Chemical Formula 1]

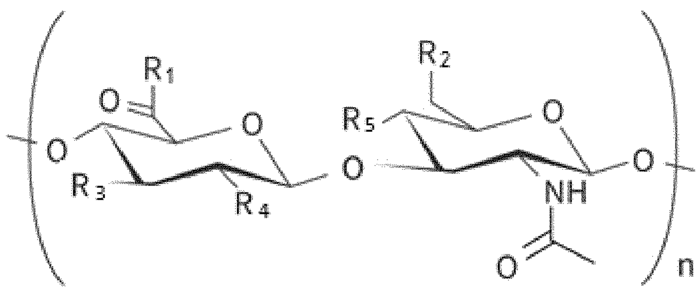

··· (1)

wherein, in the general formula (1), $R_1$, $R_2$, $R_3$, $R_4$, and $R_5$ each independently represent a hydroxy group or a group represented by $-O-CH_2-CH(OH)-CH_2-OR_6$; $R_6$ represents a linear or branched alkyl group or alkenyl group; and n represents an integer of 1 or more and 2500 or less, provided that a case where $R_1$, $R_2$, $R_3$, $R_4$, and $R_5$ are all hydroxy groups is excluded.

EP 4 523 708 A1

## Description

### Technical Field

The present invention relates to a lipid nanoparticle and a production method for the same.

### Background Art

A lipid nanoparticle is a nanoparticle having a layer formed of a lipid membrane as an outer shell. The lipid nanoparticle usually has a core capable of solubilizing a water-insoluble substance inside the layer formed of a lipid membrane. Lipid nanoparticles are used as a drug carrier in drug delivery system because a lipid nanoparticle can encapsulate a water-insoluble drug for solubilization and can encapsulate a nucleic acid that is easily degraded in vivo (for example, mRNA and siRNA) to prevent degradation thereof. For example, a lipid nanoparticle encapsulating mRNA of the spike protein of COVID-19 is used as a COVID-19 vaccine.
In order to selectively deliver a lipid nanoparticle to specific cells, the surface of the lipid nanoparticle has been modified. For example, Patent Literature 1 discloses, in order to improve the efficiency of incorporation into cells having CD44 (cancer cells and the like), a technique that a lipid nanoparticle includes a hyaluronic acid derivative in which a neutral lipid having a linear hydrophobic group is directly or indirectly linked to a hydroxyl group of N-acetylglucosamine, which is a reducing sugar at the terminal of hyaluronic acid. For example, Patent Literature 2 discloses, in order to adjust or facilitate incorporation of lipid nanoparticles into target cells, a technique that the surface of a lipid nanoparticle is coated with glycosaminoglycan such as hyaluronic acid.

### Citation List

### Patent Literature

Patent Literature 1: Japanese Unexamined Patent Publication No. 2019-189574

Patent Literature 2: Japanese Unexamined Patent Publication No. 2020-530778

### Summary of Invention

### Technical Problem

A lipid nanoparticle usually includes a phospholipid, cholesterol, and a lipid modified with polyethylene glycol (PEG). A lipid nanoparticle encapsulating a nucleic acid usually includes, in addition to these, a cationic lipid that enables complexation with a negatively charged nucleic acid molecule (such as an mRNA molecule).
The PEG-modified lipid is an important component that suppresses particle size increase due to coalescence and aggregation of lipid nanoparticles and contributes to improving the stability of a lipid nanoparticle in formulation and blood. However, the PEG-modified lipid, potentially causing allergic reactions, may cause local allergic reactions (for example, Moderna arm) or more severe systemic allergic reactions. Therefore, lipid nanoparticles as a drug carrier are required to have more options, such as a lipid nanoparticle made of alternative materials.
Patent Literatures 1 and 2 disclose that hyaluronic acid is utilized to improve incorporation of lipid nanoparticles into target cells, but do not disclose improving the stability of lipid nanoparticles.
An object of the present invention is to provide a novel lipid nanoparticle excellent in emulsifying capacity and emulsion stability and a production method for the same.

### Solution to Problem

The present inventors have found that the specific hyaluronic acid derivative described later has extremely low emulsifying capacity (ability to generate smaller particles) and emulsion stability (ability to suppress particle size increase during storage) as it is, but exhibits remarkably increased emulsifying capacity (ability to generate smaller particles) and emulsion stability (ability to suppress particle size increase during storage) when combined with phospholipids. In addition, the present inventors have also found that the lipid nanoparticle according to the present invention described later can highly efficiently encapsulate oils, drugs, and the like. The present invention is based on this novel finding.
That is, the present invention relates to, for example, the following inventions.

[1] A lipid nanoparticle including: a hyaluronic acid derivative; and a phospholipid, wherein the hyaluronic acid derivative is represented by a following general formula (1):

[Chemical Formula 1]

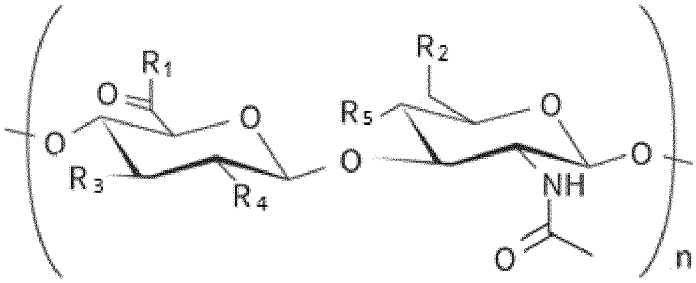

... (1)

wherein, in the general formula (1), $R_1$, $R_2$, $R_3$, $R_4$, and $R_5$ each independently represent a hydroxy group or a group represented by $-O-CH_2-CH(OH)-CH_2-OR_6$; $R_6$ represents a linear or branched alkyl group or alkenyl group; and n represents an integer of 1 or more and 2500 or less, provided that a case where $R_1$, $R_2$, $R_3$, $R_4$, and $R_5$ are all hydroxy groups is excluded.

[2] The lipid nanoparticle according to [1], wherein the phospholipid is selected from the group consisting of phosphatidylcholine, phosphatidylethanolamine, phosphatidylserine, phosphatidylglycerol, phosphatidylinositol, sphingomyelin, and a mixture of two or more thereof.

[3] The lipid nanoparticle according to [1] or [2], wherein the $R_6$ is a linear alkyl group having 8 or more and 22 or less carbon atoms.

[4] The lipid nanoparticle according to any one of [1] to [3], wherein the hyaluronic acid derivative has a modification rate of 0.1% or more and 50% or less.

[5] The lipid nanoparticle according to any one of [1] to [4], further including a substance encapsulated in an outer shell made of at least the hyaluronic acid derivative and the phospholipid.

[6] The lipid nanoparticle according to [5], wherein the substance is a water-insoluble substance.

[7] The lipid nanoparticle according to [5] or [6], wherein the substance is selected from the group consisting of a water-insoluble drug, a complex of a nucleic acid and a cationic lipid, an oil, and a mixture of two or more thereof.

[8] A production method for a lipid nanoparticle, the method including: a step of dissolving a hyaluronic acid derivative in an aqueous solvent to form an aqueous phase; a step of dissolving a phospholipid in an organic solvent to form an oil phase; and a step of dropping and dispersing the oil phase into the aqueous phase, wherein the hyaluronic acid derivative is represented by a following general formula (1):

[Chemical Formula 2]

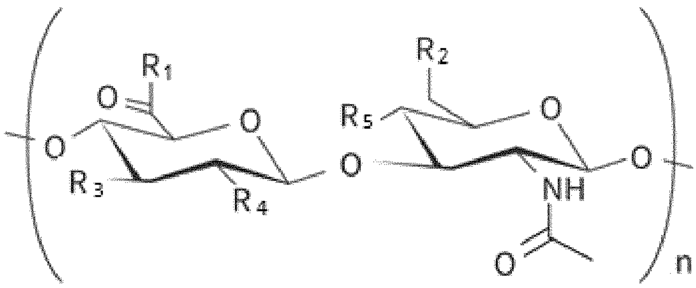

... (1)

wherein, in the general formula (1), $R_1$, $R_2$, $R_3$, $R_4$, and $R_5$ each independently represent a hydroxy group or a group represented by $-O-CH_2-CH(OH)-CH_2-OR_6$; $R_6$ represents a linear or branched alkyl group or alkenyl group; and n represents an integer of 1 or more and 2500 or less, provided that a case where $R_1$, $R_2$, $R_3$, $R_4$, and $R_5$ are all hydroxy groups is excluded.

[9] The production method according to [8], the method further including a step of subjecting a dispersion obtained in the dispersing step to a high-pressure emulsification treatment.

## Advantageous Effects of Invention

According to the present invention, it is possible to provide a novel lipid nanoparticle excellent in emulsifying capacity and emulsion stability and a production method for the same. The lipid nanoparticle of the present invention, using a specific hyaluronic acid derivative and a phospholipid in combination, is excellent in emulsifying capacity and emulsion stability. Furthermore, the lipid nanoparticle of the present invention can highly efficiently encapsulate an encapsulated substance such as oils, drugs, and the like therein, and therefore, can be used as a drug carrier in drug delivery system with substantially no PEG-modified lipid, which potentially causes allergic reactions.

## Description of Embodiments

Hereinafter, embodiments for carrying out the present invention will be described in detail. However, the present invention is not limited to the following embodiments.

<Feature of Present Invention>

(Lipid Nanoparticle)

The feature of the present invention is to provide a lipid nanoparticle including: a hyaluronic acid derivative; and a phospholipid, wherein the hyaluronic acid derivative is represented by a following general formula (1):

[Chemical Formula 3]

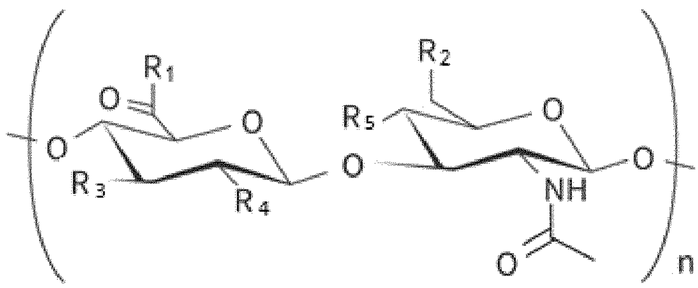

... (1)

wherein, in the general formula (1), $R_1$, $R_2$, $R_3$, $R_4$, and $R_5$ each independently represent a hydroxy group or a group represented by $-O-CH_2-CH(OH)-CH_2-OR_6$; $R_6$ represents a linear or branched alkyl group or alkenyl group; and n represents an integer of 1 or more and 2500 or less, provided that a case where $R_1$, $R_2$, $R_3$, $R_4$, and $R_5$ are all hydroxy groups is excluded.

(State of Phospholipid and Hyaluronic Acid Derivative in Lipid Nanoparticle)

The hyaluronic acid derivative included in the lipid nanoparticle of the present invention covers the lipid membrane layer formed of the phospholipid (and, if necessary, outer shell (lipid membrane layer) constituents other than a hyaluronic acid derivative such as lipids other than a phospholipid).

More specifically, the portion of the group represented by $-O-CH_2-CH(OH)-CH_2-OR_6$ in the hyaluronic acid derivative (particularly, the portion of $R_6$) is adsorbed to the lipid membrane layer as the outer shell. Accordingly, the hyaluronic acid derivative is included in the lipid nanoparticle without forming a covalent bond with the phospholipid or outer shell (lipid membrane layer) constituents other than a hyaluronic acid derivative such as lipids other than a phospholipid.

(Particle Size of Lipid Nanoparticle and Change in Particle Size after Storage)

The particle size of the lipid nanoparticle is more than 0 nm and 1000 nm or less. In the present specification, the "particle size" means an average particle size measured by a dynamic light scattering method. The particle size can be measured by a particle diameter measuring device (for example, ZETASIZER NANO, manufactured by Malvern Panalytical) using a dynamic light scattering method.

From the viewpoint of excellent emulsifying capacity, the particle size of the lipid nanoparticle of the present invention may be specifically more than 0 nm and 900 nm or less, more than 10 nm and 800 nm or less, 15 nm or more and

700 nm or less, 20 nm or more and 600 nm or less, or 25 nm or more and 500 nm or less.

Furthermore, the lipid nanoparticle of the present invention is excellent in emulsion stability. Specifically, the change from the average particle size immediately after preparation to the average particle size after storage at 40°C for 1 week is within 80 nm, and preferably within 70 nm, and further preferably within 60 nm, within 50 nm, within 40 nm, and within 35 nm. In the present invention, the average particle size of the lipid nanoparticle after storage is within the above-mentioned range.

(Production Method for Lipid Nanoparticle)

The feature of the present invention is to provide a production method for a lipid nanoparticle, the method including: a step of dissolving a hyaluronic acid derivative in an aqueous solvent to form an aqueous phase; a step of dissolving a phospholipid in an organic solvent to form an oil phase; and a step of dropping and dispersing the oil phase into the aqueous phase, wherein the hyaluronic acid derivative is represented by a following general formula (1):

[Chemical Formula 4]

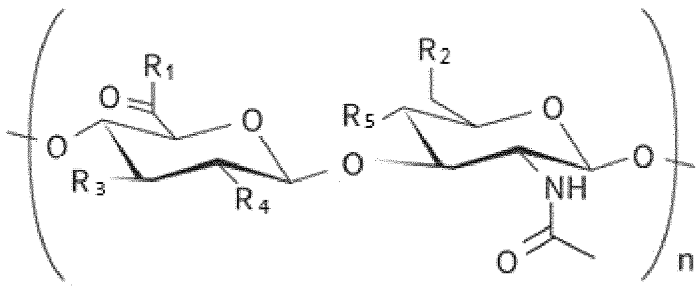

··· (1)

wherein, in the general formula (1), $R_1$, $R_2$, $R_3$, $R_4$, and $R_5$ each independently represent a hydroxy group or a group represented by $-O-CH_2-CH(OH)-CH_2-OR_6$; $R_6$ represents a linear or branched alkyl group or alkenyl group; and n represents an integer of 1 or more and 2500 or less, provided that a case where $R_1$, $R_2$, $R_3$, $R_4$, and $R_5$ are all hydroxy groups is excluded.

<Hyaluronic Acid Derivative>

In the embodiment, the hyaluronic acid derivative used in the lipid nanoparticle is represented by the following general formula (1).

[Chemical Formula 5]

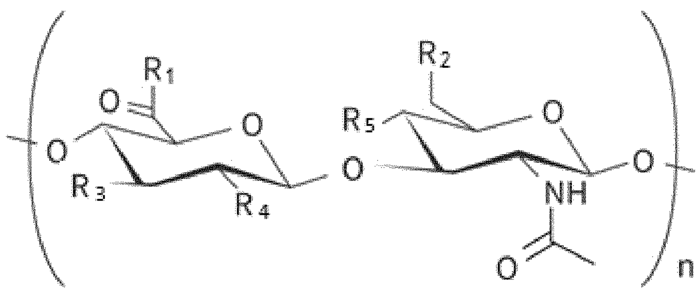

··· (1)

In the embodiment, the hyaluronic acid derivative has a structure in which some or all of hydroxyl groups bonded to the carbon atoms at the 4-position and the 6-position of N-acetylglucosamine constituting the hyaluronic acid, hydroxyl groups bonded to the carbon atoms at the 2-position and the 3-position of glucuronic acid constituting the hyaluronic acid, and a hydroxyl group in the carboxyl group bonded to the carbon atom at the 5-position are substituted with a group represented by $-O-CH_2-CH(OH)-CH_2-OR_6$. The group represented by $-O-CH_2-CH(OH)-CH_2-OR_6$ is bonded to a carbon atom constituting the hyaluronic acid via the oxygen atom described on the leftmost side.

In the general formula (1), $R_1$, $R_2$, $R_3$, $R_4$, and $R_5$ each independently represent a hydroxy group or a group represented by $-O-CH_2-CH(OH)-CH_2-OR_6$. $R_6$ represents a linear or branched alkyl group or alkenyl group. When there is a plurality of $R_1$s (when n is 2 or more), $R_1$s included in different repeating units may be the same as each other, or may be different from each other. Similarly, when there is a plurality of $R_2$s (when n is 2 or more), $R_2$s included in different repeating units may be the same as each other, or may be different from each other. When there is a plurality of $R_3$s (when n is 2 or more), $R_3$s included in different repeating units may be the same as each other, or may be different from each other. When there is a plurality of $R_4$s (when n is 2 or more), $R_4$s included in different repeating units may be the same as each other, or may be different from each other. When there is a plurality of $R_5$s (when n is 2 or more), $R_5$s included in different repeating units may be the same as each other, or may be different from each other.

Examples of the linear or branched alkyl group represented by $R_6$ include a linear or branched alkyl group having 8 or more and 22 or less carbon atoms, a linear or branched alkyl group having 9 or more and 21 or less carbon atoms, a linear or branched alkyl group having 10 or more and 20 or less carbon atoms, a linear or branched alkyl group having 11 or more and 19 or less carbon atoms, and a linear or branched alkyl group having 12 or more and 18 or less carbon atoms.

More specific examples of the linear or branched alkyl group represented by $R_6$ include a methyl group, an ethyl group, a n-propyl group, an isopropyl group, a n-butyl group, an isobutyl group, a tert-butyl group, a n-pentyl group, an isopentyl group, a neopentyl group, a tert-pentyl group, a 2-methylbutyl group, a 1-methylbutyl group, a n-hexyl group, an isohexyl group, a 3-methylpentyl group, a 2-methylpentyl group, a 1-methylpentyl group, a n-heptyl group, a n-octyl group, an isooctyl group, a 2-ethylhexyl group, a n-nonyl group, a n-decyl group, a n-undecyl group, a n-dodecyl group, a n-tridecyl group, a n-tetradecyl group (myristyl group), n-hexadecyl group (palmityl group), n-octadecyl group (stearyl group), and n-icosyl group.

Examples of the linear or branched alkenyl group represented by $R_6$ include a linear or branched alkenyl group having 8 or more and 22 or less carbon atoms, a linear or branched alkenyl group having 9 or more and 21 or less carbon atoms, a linear or branched alkenyl group having 10 or more and 20 or less carbon atoms, a linear or branched alkenyl group having 11 or more and 19 or less carbon atoms, and a linear or branched alkenyl group having 12 or more and 18 or less carbon atoms.

More specific examples of the linear or branched alkenyl group represented by $R_6$ include a vinyl group, an allyl group, a propenyl group, an isopropenyl group, a butenyl group, an isobutenyl group, a pentenyl group, an isopentenyl group, a hexenyl group, a heptenyl group, an octenyl group, a nonenyl group, a decenyl group, an undecenyl group, a dodecenyl group, a tetradecenyl group, and an oleyl group.

As the linear or branched alkyl group or alkenyl group represented by $R_6$, a linear or branched alkyl group is preferable, and a linear alkyl group is more preferable from the viewpoint of safety to a living body.

In the general formula (1), n represents an integer of 1 or more and 2500 or less. n may be, for example, 2 or more and 2500 or less, 4 or more and 2450 or less, 6 or more and 2400 or less, 8 or more and 2350 or less, 10 or more and 2300 or less, 12 or more and 2250 or less, 14 or more and 2200 or less, 16 or more and 2150 or less, 17 or more and 2100 or less, 18 or more and 2050 or less, or 19 or more and 2000 or less.

In the general formula (1), when $R_1$, $R_2$, $R_3$, $R_4$, and $R_5$ are all hydroxy groups, hyaluronic acid is obtained. Therefore, a case where $R_1$, $R_2$, $R_3$, $R_4$, and $R_5$ are all hydroxy groups is excluded from the general formula (1).

(Modification Rate of Hyaluronic Acid Derivative)

In the embodiment, for example, the hyaluronic acid derivative may have a modification rate of 0.1% or more and 50% or less. In the present specification, the modification rate of the hyaluronic acid derivative is a value in percentage indicating the number of groups represented by $-O-CH_2-CH(OH)-CH_2-OR_6$ included in one constituent unit of the hyaluronic acid. Here, the one constituent unit of the hyaluronic acid means one constituent unit made of a disaccharide of glucuronic acid and N-acetylglucosamine. The number of groups represented by $-O-CH_2-CH(OH)-CH_2-OR_6$ included in one constituent unit of the hyaluronic acid can be identified by, for example, $^1$H-NMR spectrum analysis.

From the viewpoint of enhancing the emulsion stability, the modification rate of the hyaluronic acid derivative of the present embodiment may be, for example, 0.5% or more and 45% or less, 1% or more and 40% or less, 2% or more and 30% or less, 3% or more and 20% or less, or 4% or more and 16% or less.

(Production Method of Hyaluronic Acid Derivative)

In the embodiment, the hyaluronic acid derivative can be produced, for example, by reacting hyaluronic acid or a salt thereof with a compound represented by the following general formula (2) (hereinafter, also referred to as "compound 1"). In order to enhance the reactivity, the raw material hyaluronic acid or a salt thereof may be substituted into an alkyl ammonium salt and then reacted with the compound 1.

[Chemical Formula 6]

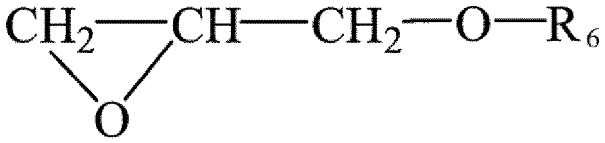

··· (2)

In the general formula (2), $R_6$ has the same meaning as $R_6$ in the general formula (1).

Examples of the raw material hyaluronic acid or a salt thereof to be used include: one extracted from a biological tissue such as an animal (for example, comb, umbilical cord, skin, joint fluid), one obtained by culturing a microorganism, an animal cell, or a plant cell (for example, a fermentation method using a bacterium of the genus Streptococcus or the like), and one chemically or enzymatically synthesized. The salt of hyaluronic acid is not particularly limited, but is preferably a food or a pharmaceutically acceptable salt. Examples thereof include a sodium salt, a potassium salt, a calcium salt, a zinc salt, a magnesium salt, an ammonium salt, and an alkylammonium salt.

The average molecular weight of the raw material hyaluronic acid or a salt thereof may be, for example, 411 or more and 1,020,000 or less. From the viewpoint of easy adsorption to the outer shell of the lipid nanoparticle and enhancing the emulsifying capacity and the emulsion stability, the average molecular weight is preferably 1000 or more and 1,000,000 or less, 2000 or more and 800,000 or less, 4000 or more and 700,000 or less, 5000 or more and 600,000 or less, 6000 or more and 500,000 or less, 7000 or more and 400,000 or less, or 8000 or more and 300,000 or less.

In the present specification, the average molecular weight of hyaluronic acid or a salt thereof is a value measured by the following method.

About 0.05 g of hyaluronic acid and/or a salt thereof (present product) is precisely weighed, and dissolved in a 0.2 mol/L sodium chloride solution to prepare an exactly 100 mL solution. Then 8 mL, 12 mL, and 16 mL of this solution are precisely weighed, and a 0.2 mol/L sodium chloride solution is added to each of them to prepare exactly 20 mL solutions. These solutions are used as sample solutions. For these sample solutions and the 0.2 mol/L sodium chloride solution, the specific viscosity is measured at 30.0 ± 0.1°C (Equation (A)) in accordance with The Japanese Pharmacopoeia, Fifteenth Edition, General Tests, Viscosity Determination, Method I Viscosity measurement by capillary tube viscometer; and then the reduced viscosity at each concentration is calculated (Equation (B)). A graph is drawn with the reduced viscosity on the vertical axis and the concentration (g/100 mL) of the present product in terms of dry product on the horizontal axis, and the limiting viscosity is determined from the intersection between the straight line connecting each point and the vertical axis. The limiting viscosity obtained here is substituted into Laurent's equation (Equation (C)) to calculate the average molecular weight (M) (Reference: T. C. Laurent, M. Ryan, A. Pietruszkiewicz,: B.B.A., 42,476-485 (1960)).

Specific viscosity = {Required flowdown number of seconds of sample solution)/(Required flowdown number of seconds of 0.2 mol/L sodium chloride solution)} -1 (Equation A):

Reduced viscosity (dL/g) = Specific viscosity/(Concentration of present product in terms of dry product g/100 mL)) (Equation B):

$$\text{(Equation C): Limiting viscosity (dL/g)} = 3.6 \times 10^{-4}\ M^{0.78}$$

(Content of Hyaluronic Acid Derivative)

In the lipid nanoparticle of the embodiment, the content of the hyaluronic acid derivative is an amount sufficient for covering the lipid membrane layer and enhancing emulsifying capacity and emulsion stability. For example, the content thereof based on the total amount of the lipid nanoparticle may be 1 mass% or more and 90 mass% or less, 1.5 mass% or more and 85 mass% or less, 2 mass% or more and 80 mass% or less, 2.5 mass% or more and 70 mass% or less, 3 mass% or more and 60 mass% or less, 3.5 mass% or more and 50 mass% or less, 4 mass% or more and 45 mass% or less, or 5 mass% or more and 40 mass% or less.

<Phospholipid>

In the embodiment, the lipid nanoparticle includes a phospholipid as a constituent component. Examples of the phospholipid include: glycerophospholipids such as phosphatidylcholine, phosphatidylethanolamine, phosphatidylserine, phosphatidylglycerol, and phosphatidylinositol; and sphingophospholipids such as sphingomyelin. The phospholipid may be used alone, or may be used in combination of two or more thereof.

As the phospholipid, for example, phospholipids derived from animal and plant raw materials such as egg yolk, soybean, and rapeseed can be used without particular limitation.

In the lipid nanoparticle of the embodiment, the content of the phospholipid is an amount sufficient for forming the lipid membrane layer. For example, the content thereof based on the total amount of the lipid nanoparticle may be 1 mass% or more and 90 mass% or less, 2 mass% or more and 85 mass% or less, 3 mass% or more and 80 mass% or less, 4 mass% or more and 70 mass% or less, 5 mass% or more and 60 mass% or less, 7 mass% or more and 50 mass% or less, 8 mass% or more and 40 mass% or less, 9 mass% or more and 30 mass% or less, or 10 mass% or more and 20 mass% or less.

In the lipid nanoparticle of the embodiment, the content ratio between the hyaluronic acid derivative and the phospholipid (content of hyaluronic acid derivative (mass%) : content of phospholipid (mass%)) may be, for example, in the range of 55 : 1 to 1 : 25, in the range of 50 : 1 to 1 : 20, or in the range of 45 : 1 to 1 : 15, from the viewpoint of enhancing emulsifying capacity and emulsion stability. When a factor that destabilizes the emulsion state is included, for example, when salts are contained as described later, the content ratio of the hyaluronic acid derivative is preferably high in order to enhance the emulsion stability.

<Encapsulated Substance>

In the embodiment, the lipid nanoparticle has a core inside the outer shell made of at least the hyaluronic acid derivative and the phospholipid, and the core may include a substance (encapsulated substance). The outer shell made of at least the hyaluronic acid derivative and the phospholipid is constituted by a lipid membrane layer formed of the portion of the group represented by $-O-CH_2-CH(OH)-CH_2-OR_6$ in the hyaluronic acid derivative (particularly, the portion of $R_6$) and the fatty acid portion of the phospholipid. When the outer phase of the lipid nanoparticle is made of a carrier mainly containing an aqueous solvent, the hydrophilic portions of the hyaluronic acid derivative and the phospholipid are arranged on the surface of the outer shell, the inside of the outer shell becomes a hydrophobic environment, and a water-insoluble substance can be suitably encapsulated. When the outer phase of the lipid nanoparticle is made of a carrier mainly containing an organic solvent, the hydrophobic portions of the hyaluronic acid derivative and the phospholipid are arranged on the surface of the outer shell, the inside of the outer shell becomes a hydrophilic environment, and a water-soluble substance can be suitably encapsulated.

The kind of the encapsulated substance is not particularly limited, and may be either a water-insoluble substance or a water-soluble substance. The water-insoluble substance means a substance having low solubility in water, and specifically means a substance whose solubility, in accordance with The Japanese Pharmacopoeia, Sixteenth Edition, is "Sparingly soluble" (the amount of solvent required to dissolve 1 g or 1 mL of solute is 30 mL or more and less than 100 mL), "Slightly soluble" (the amount of solvent as above is 100 mL or more and less than 1000 mL), "Very slightly soluble" (the amount of solvent as above is 1000 mL or more and less than 10000 mL), or "Practically insoluble" (the amount of solvent as above is 10000 mL or more).

The water-insoluble substance may be a substance having physiological activity (water-insoluble drug). Examples of the water-insoluble substance include docetaxel, paclitaxel, capecitabine, oxaliplatin, geftinut, doxorubicin, irinotecan, gemcitabine, pemetrexed, temozolomide, imatinib, vinorelbine, letrozole, teniposide, etoposide, podophyllotoxin, camptothecin, 10-hydroxycamptothecin, 9-hydroxycamptothecin, 7-ethyl-10-hydroxycamptothecin, topotecan, irinotecan, vinblastine, vincristine, vindesine, vinflunin, vinpocetine, norcantalidine, silivine, propofol, florphenicol, mitiglinide, artemisinin, dihydroartemisinin, sirolimus, ibuprofen, nitrenipin, nicardipine, nimodipine, levamipide, glyclazide, proparsid, felodipine, glibenclamide, aciclovir, oleanolic acid, bleviscapine, ferulic acid, paracetamol, palmitoylrhizoxin, penchromezin, tamoxifen, navelbine, valproic acid, tacrolimus, cyclosporin A, amphotericin B, ketoconazole, domperidone, sulpyrido, fenofibrate, bezafibrate, azithromycin, itraconazole, miconazole, brimonidine, latanoprost, silybin, erythromycin, roxithromycin, rifaximin, cisapride, cyclosporin, diclofenac acid, felodipine, ibuprofen, indomethacin, acemetacin, nicardipine, nifedipine, terfenadine, theophyllin, ketoprofen, furosemide, spironolactone, dipyridamol, piroxam, mefenamic acid, trichlorothiazide, pindolol, fat-soluble vitamins (for example, vitamin A and vitamin E), carotenoids (for example, lycopene, chlorophyll, lutein, zeaxanthin, astaxanthin, fucoxanthin), polyphenols (for example, flavonols, flavanones, flavones, isoflavones, phenol carboxylic acids, anthocyanidins, hydroxycinnamic acid derivatives, ellagic acid, and the like), and coenzyme Q10. The water-insoluble substance may be used singly or in combination of two or more thereof.

The water-soluble substance may be a substance having physiological activity (water-soluble drug). Examples of the water-soluble substance include a nucleic acid, a peptide, and a low molecular weight compound. Examples of the nucleic acid include DNA, mRNA, siRNA, microRNA, aptamer, and ribozyme. The nucleic acid may be encapsulated within the lipid nanoparticle of the embodiment in the form of a water-insoluble substance that is a complex formed with a cationic lipid. The water-soluble substance may be used singly or in combination of two or more thereof.

When the lipid nanoparticle of the embodiment includes an encapsulated substance, the encapsulated substance can be efficiently encapsulated in the lipid nanoparticle. The amount of the encapsulated substance included in the lipid nanoparticle of the embodiment (represented by encapsulation rate for water-soluble substance, and represented by encapsulated substance content in lipid nanoparticle-containing composition for water-insoluble substance) may be, for example, 60 mass% or more, 65 mass% or more, 70 mass% or more, 75 mass% or more, 80 mass% or more, 85 mass% or more, or 90 mass% or more, based on the initial amount of the encapsulated substance after storage under conditions corresponding to 40°C for 1 week.

In the embodiment, the initial amount of the encapsulated substance in the lipid nanoparticle (encapsulated substance content) may be, for example, 1 mass% or more and 95 mass% or less, 1.1 mass% or more and 94 mass% or less, 1.2 mass% or more and 93 mass% or less, 1.3 mass% or more and 92 mass% or less, 1.4 mass% or more and 91.5 mass% or less, 1.5 mass% or more and 91 mass% or less, 1.6 mass% or more and 90.9 mass% or less, 2 mass% or more and 90 mass% or less, 2.5 mass% or more and 80 mass% or less, 3 mass% or more and 70 mass% or less, 3.5 mass% or more and 60 mass% or less, 4 mass% or more and 50 mass% or less, 4.5 mass% or more and 40 mass% or less, 5 mass% or more and 30 mass% or less, or 5.5 mass% or more and 20 mass% or less based on the total amount of the lipid nanoparticle.

<Carrier>

In the embodiment, the lipid nanoparticle is usually prepared as a suspension containing the lipid nanoparticle of the embodiment and a carrier. The suspension can also be referred to as a composition containing a lipid nanoparticle (lipid nanoparticle-containing composition). The carrier constitutes the outer phase of the lipid nanoparticle. Examples of the carrier include an aqueous solvent (for example, water, buffer) used for forming an aqueous phase and an organic solvent used for forming an oil phase. Examples of the buffer include phosphate buffered saline (PBS), acetate buffer, citrate buffer, borate buffer, tartrate buffer, Tris buffer, MES buffer, and HEPES buffer. Examples of the water include Distilled Water, Water, Purified Water, Sterile Purified Water, Water for Injection, and Distilled Water for Injection, each of which is specified in The Japanese Pharmacopoeia, Eighteenth Edition.

In the lipid nanoparticle-containing composition of the embodiment, the content of the carrier may be, for example, 0 mass% or more and less than 100 mass% based on the total amount of the lipid nanoparticle-containing composition.

<Other Components>

The lipid nanoparticle-containing composition of the embodiment may further contain a food or other pharmaceutically acceptable components as long as the effect of the present invention is not inhibited. Examples of other components include a lipid other than a phospholipid, oil and fat, an organic solvent, a saccharide, a salt, a pH adjuster, and a buffer.

(Lipid other than Phospholipid)

Examples of the lipid other than a phospholipid include a sterol lipid such as cholesterol and a cholesterol derivative (for example, an ester of cholesterol and fatty acids), and a cationic lipid such as oleylamine and 3,5-bis (dodecyloxy) benzylamine. The sterol lipid contributes to forming the structure of the lipid nanoparticle (lipid membrane layer), for example. The cationic lipid is used, for example, when a negatively charged substance such as a nucleic acid is used as the encapsulated substance. The cationic lipid forms a complex with the substance to assist the solubilization of the encapsulated substance.

(Oil and Fat)

The oil and fat is used, for example, to assist the solubilization of the encapsulated substance. Examples of the oil and fat include a vegetable oil such as soybean oil, sesame oil, rapeseed oil, safflower oil, olive oil, castor oil, corn oil, cottonseed oil, rice oil, sunflower oil, grape seed oil, and wheat germ oil, medium-chain fatty acid triglyceride (MCT), and long-chain fatty acid triglyceride (LCT).

(Saccharide)

The saccharide is used, for example, to adjust the osmotic pressure. Examples of the saccharide include sorbitol, xylitol, mannitol, glucose, trehalose, maltose, sucrose, raffinose, lactose, and dextran.

(Salt)

Salt is used, for example, to improve the encapsulation rate of the encapsulated substance (for example, nucleic acid) or to adjust the osmotic pressure. Examples of the salt include sodium chloride and potassium chloride. The salt is a causative substance for lowering the emulsion stability of the lipid nanoparticle. However, the lipid nanoparticle of the embodiment is excellent in emulsifying capacity and emulsion stability. Therefore, the emulsion stability thereof can be maintained even when the salt is contained. Specifically, in the lipid nanoparticle of the embodiment, the content of the salt (for example, sodium chloride) may be, for example, 0 mass% or more and 1500 mass% or less, 0 mass% or more and 1400 mass% or less, 0 mass% or more and 1300 mass% or less, 0 mass% or more and 1200 mass% or less, or 0 mass% or more and 1156 mass% or less, based on the total amount of the lipid nanoparticle. Here, the content of the salt is an amount including salts present in the outer phase.

(pH Adjuster)

Examples of the pH adjuster include a strong acid such as hydrochloric acid and a strong base such as sodium hydroxide.

(Buffer)

Examples of the buffer include a phosphate buffer, a borate buffer, and a Tris buffer.

<Stabilizer>

The lipid nanoparticle of the embodiment, using the hyaluronic acid derivative and the phospholipid in combination, is excellent in emulsion stability. In addition, the hyaluronic acid derivative and the phospholipid are excellent in safety for living bodies, and are less likely to cause allergic reactions. Therefore, the lipid nanoparticle of the embodiment may contain substantially no stabilizer potentially causing allergic reactions. Examples of the stabilizer potentially causing allergic reactions include polyethylene glycol (including free form and a form of bonding to lipids and the like). The phrase "contain substantially no stabilizer potentially causing allergic reactions" means that the content of the stabilizer potentially causing allergic reactions is 5 mass% or less based on the total amount of the lipid nanoparticle, and may be 4 mass% or less, 3 mass% or less, 2 mass% or less, 1 mass% or less, or 0 mass% or less.

<Lipid Nanoparticle>

(Form)

The form of the lipid nanoparticle-containing composition of the embodiment is not particularly limited, and the lipid nanoparticle-containing composition can be prepared in any form according to the intended use. Specifically, examples of the form thereof include a suspension containing a carrier, and a powder obtained by freeze-drying the suspension as it is or after the carrier is removed.

(Application)

The lipid nanoparticle of the embodiment can be used, for example, as a drug carrier encapsulating a physiologically active substance inside the outer shell (lipid membrane layer). Specifically, the lipid nanoparticle of the embodiment can be used as, for example, an injection, an eye drop, a fat emulsion, or the like.

(Administration form)

The lipid nanoparticle of the embodiment can be administered by injection, and can also be administered by spraying, dipping, rinsing, application from a pad or a ball roller, painting, dropping, aerosol spray, or pump spray.

(Administration Target)

The target to which the lipid nanoparticle of the embodiment is administered may be, for example, a mammal such as a human.

(Administration dose)

The dose, administration timing, and administration period of the lipid nanoparticle of the embodiment can be determined according to the type of the physiologically active substance to be used, the type of the administration target, and the like.

<Production Method for Lipid Nanoparticle>

The production method for a lipid nanoparticle of the embodiment includes: a step of dissolving a hyaluronic acid derivative in an aqueous solvent to form an aqueous phase (aqueous phase forming step); a step of dissolving a phospholipid in an organic solvent to form an oil phase (oil phase forming step); and a step of dropping and dispersing the oil phase into the aqueous phase (dispersing step). The production method for a lipid nanoparticle of the embodiment may further include, in addition to the steps, a step of subjecting a dispersion obtained in the dispersing step to a high-pressure emulsification treatment (fine emulsification step).

(Aqueous Phase Forming Step)

In the aqueous phase forming step, a hyaluronic acid derivative is dissolved in an aqueous solvent to form an aqueous phase. Water-soluble components other than the hyaluronic acid derivative are preferably dissolved in the aqueous solvent together with the hyaluronic acid derivative in the aqueous phase forming step.

Examples of the aqueous solvent include water and a buffer. Examples of the buffer include phosphate buffered saline (PBS), acetate buffer, citrate buffer, borate buffer, tartrate buffer, Tris buffer, MES buffer, and HEPES buffer.

The method for dissolving the hyaluronic acid derivative and, if necessary, water-soluble components other than the hyaluronic acid derivative in the aqueous solvent is not particularly limited. For example, they can be dissolved by stirring or the like. In addition, if necessary, they may be dissolved by heating at 40 to 80°C.

(Oil Phase Forming Step)

In the oil phase forming step, a phospholipid is dissolved in an organic solvent to form an oil phase. Water-insoluble components other than the phospholipid are preferably dissolved in the organic solvent together with the phospholipid in the oil phase forming step.

Examples of the organic solvent include ethanol, dimethylformamide (DMF), propylene glycol, benzene, acetone, dimethylformamide, dimethyl sulfoxide, N-methyl-2-pyrrolidinone, tetrahydrofuran, and chloroform.

The method for dissolving the phospholipid and, if necessary, water-insoluble components other than the phospholipid in the organic solvent is not particularly limited. For example, they can be dissolved by stirring or the like. In addition, if necessary, they may be dissolved by heating at 40 to 80°C.

In the production method of the embodiment, the aqueous phase forming step and the oil phase forming step may be performed in any order. For example, the aqueous phase forming step may be performed first, the oil phase forming step may be performed first, and the aqueous phase forming step and the oil phase forming step may be performed simultaneously.

(Dispersing Step)

In the dispersing step, the oil phase is dropped and dispersed into the aqueous phase. By performing the dispersing step, the lipid nanoparticle of the embodiment is obtained. The oil phase is dispersed, for example, by gently dropping the oil phase into the aqueous phase at a constant rate, and then heating and stirring at 50 to 70°C and 5,000 to 15,000 rpm for 1 to 30 minutes. The particle size of the lipid nanoparticle obtained in the dispersing step is usually in the range of 1 nm to 500 nm.

By performing the dispersing step described above, the hydrophilic portions of the hyaluronic acid derivative and the phospholipid are arranged on the surface of the outer shell, the inside of the outer shell becomes a hydrophobic environment, and a water-insoluble substance can be suitably encapsulated. When the inside of the outer shell becomes a hydrophilic environment, for example, the aqueous phase is dropped and dispersed into the oil phase in the dispersing step.

(Fine Emulsification Step)

In the fine emulsification step, the dispersion obtained in the dispersing step is subjected to a high-pressure emulsification treatment. Also by performing the fine emulsification step, the lipid nanoparticle of the embodiment is

obtained. The high-pressure emulsification treatment can be performed using, for example, a high pressure emulsifier. The high-pressure emulsification treatment can be performed, for example, by passing liquid 3 to 30 times at a pressure of 50 to 200 MPa under a temperature condition of 50 to 70°C. Examples of the high pressure emulsifier include chamber type high pressure homogenizers such as MICROFLUIDIZER (manufactured by MICROFLUIDICS), NANOMIZER (manufactured by YOSHIDA KIKAI CO., LTD.), and STAR BURST (manufactured by Sugino Machine Limited); and homogeneous valve type high pressure homogenizers such as Gaulin type homogenizer (manufactured by APV), Rannie type homogenizer (manufactured by Rannie), high pressure homogenizer (manufactured by Niro Soavi), homogenizer (manufactured by SANWA MACHINERY TRADING CO., LTD.), high pressure homogenizer (manufactured by IZUMI FOOD MACHINERY Co., Ltd.), and ultra high pressure homogenizer (manufactured by IKA). The particle size of the lipid nanoparticle obtained in the fine emulsification step is usually in the range of 1 nm to 250 nm.

(Other Steps)

The production method for a lipid nanoparticle of the embodiment may further include, in addition to the above steps, a sterilization step and a pH adjustment step as necessary.

(Sterilization Step)

In the sterilization step, the lipid nanoparticle obtained in the dispersing step or the fine emulsification step is sterilized. The lipid nanoparticle is sterilized in accordance with a conventional method. Specifically, for example, the lipid nanoparticle can be sterilized by passing through a membrane filter (for example, a nylon syringe filter) having a pore size of 0.2 to 0.22 μm.

(pH Adjustment Step)

In the pH adjustment step, a pH adjuster is mixed to adjust the pH of the lipid nanoparticle. The pH adjustment step may be performed as necessary, and is not an essential step. The pH adjustment step is preferably performed before the sterilization step. Specifically, for example, the pH adjustment step can be performed by further adding a pH adjuster in the aqueous phase forming step, further adding a pH adjuster in the oil phase forming step, further adding a pH adjuster in the dispersing step, further adding a pH adjuster in the fine emulsification step, or the like. The addition amount of the pH adjuster can be determined so that the pH of the finally obtained lipid nanoparticle falls within a desired range.

## Examples

Hereinafter, the present invention will be described more specifically based on examples and the like. However, the present invention is not limited to the following examples.

The correspondence between the components used in examples and the abbreviations thereof is as follows.

MCT: COCONARD RK (trade name), (glyceryl tricaprylate), manufactured by Kao Corporation
PC: PC-98T (trade name), manufactured by Kewpie Corporation (egg yolk lecithin purified to have a phosphatidylcholine content of 98% or more)
PBS: Phosphate buffered saline (pH 7.4), manufactured by NACALAI TESQUE, INC.
CH: Cholesterol, manufactured by Tokyo Chemical Industry Co., Ltd. (cholesterol)
CHE: Cholesterol Oleate, manufactured by Tokyo Chemical Industry Co., Ltd. (oleic ester of cholesterol)
OA: Oleic acid, manufactured by Tokyo Chemical Industry Co., Ltd.
CsA: Cyclosporin A, manufactured by LKT Laboratories
Tac: Tacrolimus hydrate, manufactured by Tokyo Chemical Industry Co., Ltd.
PL: PL-100M (trade name), Kewpie Corporation (Egg yolk lecithin containing 80% phosphatidylcholine and 20% phosphatidylethanolamine)
LCT: Japanese Pharmacopoeia soybean oil, manufactured by Kao Corporation (long-chain fatty acid triglyceride in which fatty acid has mainly 18 carbon atoms)
RD: Rebamipide, manufactured by Tokyo Chemical Industry Co., Ltd.
NP: Nimodipine, manufactured by Tokyo Chemical Industry Co., Ltd.

[Test Example 1: Production of Hyaluronic Acid Derivative and Evaluation of Emulsifying Capacity and Emulsion Stability]

<Production of Hyaluronic Acid Derivative>

A hyaluronic acid derivative was produced by the method described in Example 1 of Japanese Patent No. 4845071. Specifically, 5.0 g of hyaluronic acid (average molecular weight 8000, manufactured by Kewpie Corporation) was dissolved in 500 mL of water in a 1 L beaker, and then a 40 w/v% aqueous tetrabutylammonium hydroxide solution was added thereto while stirring to adjust the pH to 7.2. After adjusting the pH, the mixture was freeze-dried to obtain 10.2 g of a tetrabutylammonium salt of hyaluronic acid. In a 30 mL sample bottle, 1.0 g of the obtained tetrabutylammonium salt of hyaluronic acid, 2.0 g of $C_{12\ to\ 13}$ alkyl glycidyl ether (reaction reagent) (manufactured by Yokkaichi Chemical Company Limited), and 10 mL of dimethylformamide (DMF) were charged, and the mixture was reacted in an 80°C water bath for 8 hours while being stirred. After completion of the reaction, 10 mL of a 12.5 w/v% aqueous sodium chloride solution was added thereto, and the mixture was adjusted to pH 1.0 with 8 w/v% hydrochloric acid. Next, 50 mL of ethanol was then added slowly while stirring to precipitate hyaluronic acid. Then, the pH was adjusted to 7.0 with 25 w/v% sodium hydroxide, and the precipitate was collected by filtration and washed three times with 50 mL of an 80 v/v% ethanol aqueous solution. The obtained precipitate was vacuum-dried at 60°C to obtain 0.48 g of a hyaluronic acid derivative represented by the following general formula (1).

[Chemical Formula 7]

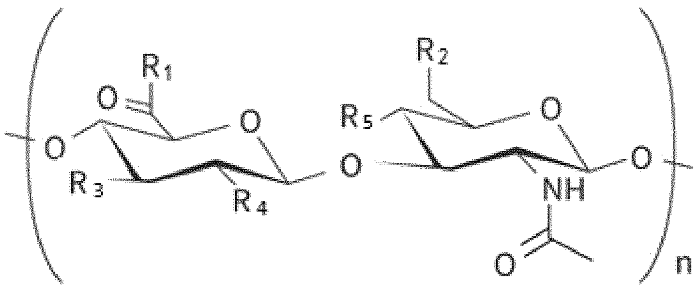

... (1)

In the general formula (1), $R_1$, $R_2$, $R_3$, $R_4$, and $R_5$ each independently represent a hydroxy group or a group represented by $-O-CH_2-CH(OH)-CH_2-OR_6$.

In the hyaluronic acid derivative produced in Test Example 1, $R_6$ represents an alkyl group having 12 or 13 carbon atoms, and n represents an integer of 1 to 50.

<Production of Lipid Nanoparticle>

A lipid nanoparticle encapsulating MCT as a water-insoluble substance was prepared by the following method.

PC and MCT were dissolved in ethanol in an amount of 0.1 mass% and 0.5 mass%, respectively, to obtain an ethanol solution. While a solution in which 0.5 mg of the hyaluronic acid derivative had been dissolved in 4.5 mL of PBS was being vigorously stirred, 0.5 mL of the ethanol solution was added dropwise to prepare a lipid nanoparticle encapsulating MCT (Example 1). Besides, a lipid nanoparticle encapsulating MCT was prepared in the same manner as in Example 1 except that no PC was contained in the ethanol solution (Comparative Example 1).

<Evaluation of Particle Size>

The particle size of each of the lipid nanoparticles of Example 1 and Comparative Example 1 was measured immediately after preparation and after storage at 40°C for 1 week. The particle size was measured by a particle diameter measuring device (ZETASIZER NANO, manufactured by Malvern Panalytical) using a dynamic light scattering method. The results are shown in Table 1. In the example, the phospholipid content, the salt content, the encapsulated substance content, and the hyaluronic acid derivative content are values calculated from the formulation, and are based on the total amount of the lipid nanoparticle. Here, PBS contains 0.9 mass% of sodium chloride.

[Table 1]

| | | Comparative Example 1 | Example 1 |
|---|---|---|---|
| PC (mass%) | | - | 0.01 |
| MCT (mass%) | | 0.05 | 0.05 |
| Hyaluronic acid derivative (mass%) | | 0.01 | 0.01 |
| Ethanol (mass%) | | 10.00 | 10.00 |
| PBS (mass%) | | 89.94 | 89.93 |
| Ratio (PC : MCT : Hyaluronic acid derivative) | | 0 : 5 : 1 | 1 : 5 : 1 |
| Phospholipid content (mass%) | | - | 14.29 |
| Salt content (mass%) | | 1619.00 | 1156.00 |
| Encapsulated substance content (mass%) | | 83.00 | 71.00 |
| Hyaluronic acid derivative content (mass%) | | 16.67 | 14.29 |
| Particle size (nm) | Immediately after preparation | 255 | 159 |
| | After storage at 40°C for 1 week | 394 | 173 |
| Change in particle size (nm) after storage at 40°C for 1 week | | +139 | +14 |

From the results of the lipid nanoparticle prepared without using PC (Comparative Example 1), it is found that the hyaluronic acid derivative represented by the formula (1) itself has extremely low emulsifying capacity (ability to generate smaller particles) and emulsion stability (ability to suppress particle size increase during storage).

[Test Example 2: Evaluation of Emulsion Assisting Action of Hyaluronic Acid Derivative (pH 7.4)]

<Production of Hyaluronic Acid Derivative>

The hyaluronic acid derivative was produced in the same manner as in Test Example 1.

<Production of Lipid Nanoparticle>

A lipid nanoparticle encapsulating MCT as a water-insoluble substance was prepared by the following method.
PC, CH, and MCT were dissolved in ethanol in an amount of 0.1 mass%, 0.2 mass%, and 0.5 mass%, respectively, to obtain an ethanol solution. While a solution in which 5 mg of the hyaluronic acid derivative had been dissolved in 4.5 mL of PBS was being vigorously stirred, 0.5 mL of the ethanol solution was added dropwise to prepare a lipid nanoparticle encapsulating MCT (Example 2). Besides, a lipid nanoparticle encapsulating MCT was prepared in the same manner as in Example 2 except that no hyaluronic acid derivative was contained in PBS (Comparative Example 2).

<Evaluation of Particle Size>

The particle size of each of the lipid nanoparticles of Example 2 and Comparative Example 2 was measured immediately after preparation and after storage at 40°C for 1 week. The particle size was measured by a particle diameter measuring device (ZETASIZER NANO, manufactured by Malvern Panalytical) using a dynamic light scattering method. The results are shown in Table 2.

[Table 2]

| | Comparative Example 2 | Example 2 |
|---|---|---|
| PC (mass%) | 0.01 | 0.01 |
| CH (mass%) | 0.02 | 0.02 |
| MCT (mass%) | 0.05 | 0.05 |
| Hyaluronic acid derivative (mass%) | - | 0.10 |
| Ethanol (mass%) | 10.00 | 10.00 |

(continued)

| | | Comparative Example 2 | Example 2 |
|---|---|---|---|
| PBS (mass%) | | 89.92 | 89.82 |
| Ratio (PC : MCT : Hyaluronic acid derivative) | | 1 : 5 : 0 | 1 : 5 : 10 |
| Phospholipid content (mass%) | | 12.50 | 5.56 |
| Salt content (mass%) | | 1012.00 | 449.00 |
| Encapsulated substance content (mass%) | | 63.00 | 28.00 |
| Hyaluronic acid derivative content (mass%) | | - | 55.56 |
| Particle size (nm) | Immediately after preparation | 229 | 157 |
| | After storage at 40°C for 1 week | 325 | 189 |
| Change in particle size (nm) after storage at 40°C for 1 week | | +96 | +32 |

It has been found that, when the hyaluronic acid derivative represented by the formula (1) is added to the lipid nanoparticle mainly including a phospholipid, emulsifying capacity (ability to generate smaller particles) and emulsion stability (ability to suppress particle size increase during storage) are remarkably increased. That is, it can be found that the hyaluronic acid derivative represented by the general formula (1) is excellent in emulsion assisting action.

[Test Example 3: Evaluation of Emulsion Assisting Action of Hyaluronic Acid Derivative (pH 4.0)]

<Production of Hyaluronic Acid Derivative>

The hyaluronic acid derivative was produced in the same manner as in Test Example 1.

<Production of Lipid Nanoparticle>

A lipid nanoparticle encapsulating MCT as a water-insoluble substance was prepared by the following method.

PC, CH, and MCT were dissolved in ethanol in an amount of 0.125 mass%, 0.25 mass%, and 0.625 mass%, respectively, to obtain an ethanol solution. While a solution in which 5 mg of the hyaluronic acid derivative had been dissolved in 5 mL of 100 mM acetate buffer (pH 4.0) was being vigorously stirred, 0.5 mL of the ethanol solution was added dropwise to prepare a lipid nanoparticle encapsulating MCT (Example 3). Besides, a lipid nanoparticle encapsulating MCT was prepared in the same manner as in Example 3 except that no hyaluronic acid derivative was contained in 100 mM acetate buffer (Comparative Example 3).

<Evaluation of Particle Size>

The particle size of each of the lipid nanoparticles of Example 3 and Comparative Example 3 was measured immediately after preparation and after storage at normal temperature for 1 day. The particle size was measured by a particle diameter measuring device (ZETASIZER NANO, manufactured by Malvern Panalytical) using a dynamic light scattering method. The results are shown in Table 3. Here, the 100 mM acetate buffer contains 0.18 mass% of sodium acetate.

[Table 3]

| | Comparative Example 3 | Example 3 |
|---|---|---|
| PC (mass%) | 0.011 | 0.011 |
| CH (mass%) | 0.023 | 0.023 |
| MCT (mass%) | 0.057 | 0.057 |
| Hyaluronic acid derivative (mass%) | - | 0.100 |
| Ethanol (mass%) | 9.091 | 9.091 |
| 100 mM acetate buffer (mass%) | 90.82 | 90.72 |

(continued)

| | | Comparative Example 3 | Example 3 |
|---|---|---|---|
| Ratio (PC : MCT : Hyaluronic acid derivative) | | 1 : 5 : 0 | 1 : 5 : 10 |
| Phospholipid content (mass%) | | 12.09 | 5.76 |
| Salt content (mass%) | | 179.66 | 85.50 |
| Encapsulated substance content (mass%) | | 62.64 | 29.84 |
| Hyaluronic acid derivative content (mass%) | | - | 52.36 |
| Particle size (nm) | Immediately after preparation | 275 | 131 |
| | After storage at normal temperature for 1 day | 386 | 146 |
| Change in particle size (nm) after storage at normal temperature for 1 day | | +111 | +15 |

Similarly to Test Example 2, even when the pH differs, it has been found that, when the hyaluronic acid derivative represented by the formula (1) is added to the lipid nanoparticle mainly including a phospholipid, emulsifying capacity (ability to generate smaller particles) and emulsion stability (ability to suppress particle size increase during storage) are remarkably increased.

[Test Example 4: Evaluation of Emulsion Assisting Action of Hyaluronic Acid Derivative (Freeze-thaw resistance)]

<Production of Hyaluronic Acid Derivative>

The hyaluronic acid derivative was produced in the same manner as in Test Example 1.

<Production of Lipid Nanoparticle>

A lipid nanoparticle encapsulating MCT as a water-insoluble substance was prepared by the following method.

PC, CH, and MCT were dissolved in ethanol in an amount of 0.125 mass%, 0.25 mass%, and 0.625 mass%, respectively, to obtain an ethanol solution. While a solution in which 5 mg of the hyaluronic acid derivative had been dissolved in 5 mL of PBS was being vigorously stirred, 0.5 mL of the ethanol solution was added dropwise to prepare a lipid nanoparticle encapsulating MCT (Example 4). Besides, a lipid nanoparticle encapsulating MCT was prepared in the same manner as in Example 4 except that no hyaluronic acid derivative was contained in PBS (Comparative Example 4).

<Evaluation of Particle Size>

The lipid nanoparticles of Example 4 and Comparative Example 4 were freeze-thawed. That is, each lipid nanoparticle was cooled to - 40°C for rapid freezing, and then thawed at normal temperature. Next, the particle size of each of the lipid nanoparticles of Example 4 and Comparative Example 4 was measured immediately after preparation and after freeze-thaw. The particle size was measured by a particle diameter measuring device (ZETASIZER NANO, manufactured by Malvern Panalytical) using a dynamic light scattering method. The results are shown in Table 4.

[Table 4]

| | Comparative Example 4 | Example 4 |
|---|---|---|
| PC (mass%) | 0.011 | 0.011 |
| CH (mass%) | 0.023 | 0.023 |
| MCT (mass%) | 0.057 | 0.057 |
| Hyaluronic acid derivative (mass%) | - | 0.100 |
| Ethanol (mass%) | 9.091 | 9.091 |
| PBS (mass%) | 90.82 | 90.72 |
| Ratio (PC : MCT : Hyaluronic acid derivative) | 1 : 5 : 0 | 1 : 5 : 10 |

(continued)

| | | Comparative Example 4 | Example 4 |
|---|---|---|---|
| Phospholipid content (mass%) | | 12.09 | 5.76 |
| Salt content (mass%) | | 898.31 | 427.52 |
| Encapsulated substance content (mass%) | | 62.64 | 29.84 |
| Hyaluronic acid derivative content (mass%) | | - | 52.36 |
| Particle size (nm) | Immediately after preparation | 235 | 119 |
| | After freeze-thaw | 449 | 121 |
| Change in particle size (nm) after freeze-thaw | | +214 | +2 |

It has been found that, when the hyaluronic acid derivative represented by the formula (1) is added to the lipid nanoparticle mainly including a phospholipid, emulsion stability (ability to suppress particle size increase during storage) during freeze-thaw is remarkably increased. In addition, pharmaceutical products that are easily decomposed are usually cryopreserved. However, it is difficult to cryopreserve a lipid nanoparticle mainly including a phospholipid. On the other hand, the lipid nanoparticle of the present invention can also be cryopreserved.

[Test Example 5: Variation Evaluation of Hyaluronic Acid Derivative]

<Production of Hyaluronic Acid Derivative>

(Hyaluronic Acid Derivative A)

The hyaluronic acid derivative A was produced in the same manner as in Test Example 1. The hyaluronic acid derivative A has an average molecular weight of 8,000 as the raw material hyaluronic acid, and $R_6$ in the general formula (1) is an alkyl group having 12 or 13 carbon atoms.

(Hyaluronic Acid Derivative B)

The hyaluronic acid derivative B was produced in the same manner as in Test Example 1 except that $C_{18}$ alkyl glycidyl ether was used instead of $C_{12\ to\ 13}$ alkyl glycidyl ether. The hyaluronic acid derivative B has an average molecular weight of 8,000 as the raw material hyaluronic acid, and $R_6$ in the general formula (1) is an alkyl group having 18 carbon atoms.

(Hyaluronic Acid Derivative C)

The hyaluronic acid derivative C was obtained in the same manner as in Test Example 1 except that a high-molecular hyaluronic acid (average molecular weight 100,000, manufactured by Kewpie Corporation) was used instead of the hyaluronic acid (average molecular weight 8,000, manufactured by Kewpie Corporation). The hyaluronic acid derivative C has an average molecular weight of 100,000 as the raw material hyaluronic acid, and $R_6$ in the general formula (1) is an alkyl group having 12 or 13 carbon atoms.

<Production of Lipid Nanoparticle>

A lipid nanoparticle encapsulating MCT as a water-insoluble substance was prepared by the following method.

PC, CH, and MCT were dissolved in ethanol in an amount of 0.1 mass%, 0.2 mass%, and 0.5 mass%, respectively, to obtain an ethanol solution.

While a solution in which 5 mg of the hyaluronic acid derivative A had been dissolved in 4.5 mL of PBS was being vigorously stirred, 0.5 mL of the ethanol solution was added dropwise to prepare a lipid nanoparticle encapsulating MCT (Example 7). A lipid nanoparticle encapsulating MCT was prepared in the same manner as in Example 7, except that 2.5 mg of the hyaluronic acid (average molecular weight 8,000, manufactured by Kewpie Corporation) and 2.5 mg of the hyaluronic acid derivative A were used instead of 5 mg of the hyaluronic acid derivative A (Example 6). Furthermore, a lipid nanoparticle encapsulating MCT was prepared in the same manner as in Example 7, except that 4 mg of the hyaluronic acid (average molecular weight 8,000, manufactured by Kewpie Corporation) and 1 mg of the hyaluronic acid derivative A were used instead of 5 mg of the hyaluronic acid derivative A (Example 5). Furthermore, a lipid nanoparticle encapsulating

MCT was prepared in the same manner as in Example 7, except that 5 mg of the hyaluronic acid (average molecular weight 8,000, manufactured by Kewpie Corporation) was used instead of 5 mg of the hyaluronic acid derivative A (Comparative Example 5). Comparative Example 5, Example 5, Example 6, and Example 7 correspond to cases where the modification rate of the hyaluronic acid derivative A is changed to 0%, 1%, 2.5%, and 5%, respectively. The modification rate of each of the hyaluronic acid derivative A, the hyaluronic acid derivative B, and the hyaluronic acid derivative C was measured by $^1$H-NMR spectrum analysis.

While a solution in which 5 mg of the hyaluronic acid derivative B or 5 mg of the hyaluronic acid derivative C had been dissolved in 4.5 mL of PBS was being vigorously stirred, 0.5 mL of the ethanol solution was added dropwise to prepare a lipid nanoparticle encapsulating MCT (Examples 8 and 9).

<Evaluation of Particle Size>

The particle size of each of the lipid nanoparticles of Comparative Example 5 and Examples 5 to 9 was measured immediately after preparation and after storage at normal temperature for 1 day. The particle size was measured by a particle diameter measuring device (ZETASIZER NANO, manufactured by Malvern Panalytical) using a dynamic light scattering method. The results are shown in Table 5.

[Table 5]

| | Comparative Example 5 | Example 5 | Example 6 | Example 7 | Example 8 | Example 9 |
|---|---|---|---|---|---|---|
| PC (mass%) | 0.010 | 0.010 | 0.010 | 0.010 | 0.010 | 0.010 |
| CH (mass%) | 0.020 | 0.020 | 0.020 | 0.020 | 0.020 | 0.020 |
| MCT (mass%) | 0.050 | 0.050 | 0.050 | 0.050 | 0.050 | 0.050 |
| Hyaluronic acid (mass%) | 0.010 | 0.008 | 0.005 | - | - | - |
| Hyaluronic acid derivative A (mass%) | - | 0.002 | 0.005 | 0.010 | - | - |
| Hyaluronic acid derivative B (mass%) | - | - | - | - | 0.010 | - |
| Hyaluronic acid derivative C (mass%) | - | - | - | - | - | 0.010 |
| Ethanol (mass%) | 10.00 | 10.00 | 10.00 | 10.00 | 10.00 | 10.00 |
| PBS (mass%) | 89.91 | 89.91 | 89.91 | 89.91 | 89.91 | 89.91 |
| Ratio (PC : MCT : Hyaluronic acid derivative) | 1 : 5 : 1 | 1 : 5 : 1 | 1 : 5 : 1 | 1 : 5 : 1 | 1 : 5 : 1 | 1 : 5 : 1 |
| Average molecular weight of raw material hyaluronic acid | 8000 | 8000 | 8000 | 8000 | 8000 | 100000 |
| Number of carbon atoms in alkyl group represented by $R_6$ | 12 to 13 | 12 to 13 | 12 to 13 | 12 to 13 | 18 | 12 to 13 |
| Modification rate of hyaluronic acid derivative (%) | 0 | 1 | 2.5 | 5 | 8 | 16 |
| Phospholipid content (mass%) | 11.11 | 11.11 | 11.11 | 11.11 | 11.11 | 11.11 |
| Salt content (mass%) | 899.10 | 899.10 | 899.10 | 899.10 | 899.10 | 899.10 |
| Encapsulated substance content (mass%) | 55.56 | 55.56 | 55.56 | 55.56 | 55.56 | 55.56 |
| Hyaluronic acid derivative content (mass%) | 11.11 | 11.11 | 11.11 | 11.11 | 11.11 | 11.11 |

(continued)

| | | Comparative Example 5 | Example 5 | Example 6 | Example 7 | Example 8 | Example 9 |
|---|---|---|---|---|---|---|---|
| Particle size (nm) | Immediately after preparation | 223 | 182 | 184 | 151 | 202 | 412 |
| | After storage at 40°C for 1 week | 588 | 238 | 253 | 185 | 212 | 401 |
| Change in particle size (nm) after storage at 40°C for 1 week | | +365 | +56 | +69 | +34 | +10 | -11 |

As shown in Table 5, even when the average molecular weight of the raw material hyaluronic acid, the number of carbon atoms in alkyl group represented by $R_6$ in the formula (1), or the modification rate were changed, it has been found that, when the hyaluronic acid derivative represented by the formula (1) is added to the lipid nanoparticle mainly including a phospholipid, emulsifying capacity (ability to generate smaller particles) and emulsion stability (ability to suppress particle size increase during storage) are remarkably increased.

[Test Example 6: Variation Evaluation of Encapsulated Substance]

<Production of Hyaluronic Acid Derivative>

The hyaluronic acid derivative was produced in the same manner as in Test Example 1.

<Production of Lipid Nanoparticle>

A lipid nanoparticle encapsulating a water-insoluble substance (MCT, CHE, or OA) was prepared by the following method.

PC, CH, and a water-insoluble substance (MCT) were dissolved in ethanol in an amount of 0.1 mass%, 0.2 mass%, and 0.5 mass%, respectively, to obtain an ethanol solution. While a solution in which 5 mg of the hyaluronic acid derivative had been dissolved in 4.5 mL of PBS was being vigorously stirred, 0.5 mL of the ethanol solution was added dropwise to prepare a lipid nanoparticle encapsulating a water-insoluble substance (MCT) (Example 10). PC and a water-insoluble substance (CHE or OA) were dissolved in ethanol in an amount of 0.1 mass% and 0.5 mass%, respectively, to obtain an ethanol solution. While a solution in which 5 mg of the hyaluronic acid derivative had been dissolved in 4.5 mL of PBS was being vigorously stirred, 0.5 mL of the ethanol solution was added dropwise to prepare a lipid nanoparticle encapsulating a water-insoluble substance (CHE or OA) (Examples **11** and 12).

<Evaluation of Particle Size>

The particle size of each of the lipid nanoparticles of Examples 10 to 12 was measured immediately after preparation and after storage at 40°C for 1 week. The particle size was measured by a particle diameter measuring device (ZETASIZER NANO, manufactured by Malvern Panalytical) using a dynamic light scattering method. The results are shown in Table 6.

[Table 6]

| | Example 10 | Example 11 | Example 12 |
|---|---|---|---|
| PC (mass%) | 0.01 | 0.01 | 0.01 |
| CH (mass%) | 0.02 | - | - |
| MCT (mass%) | 0.05 | - | - |
| CHE (mass%) | - | 0.05 | - |
| OA (mass%) | - | - | 0.05 |
| Hyaluronic acid derivative (mass%) | 0.20 | 0.20 | 0.20 |

(continued)

| | | Example 10 | Example 11 | Example 12 |
|---|---|---|---|---|
| Ethanol (mass%) | | 10.00 | 10.00 | 10.00 |
| PBS (mass%) | | 89.72 | 89.74 | 89.74 |
| Ratio (PC : Encapsulated substance : Hyaluronic acid derivative) | | 1 : 5 : 20 | 1 : 5 : 20 | 1 : 5 : 20 |
| Phospholipid content (mass%) | | 3.57 | 3.85 | 3.85 |
| Salt content (mass%) | | 288.45 | 310.64 | 310.64 |
| Encapsulated substance content (mass%) | | 17.86 | 19.23 | 19.23 |
| Hyaluronic acid derivative content (mass%) | | 71.43 | 76.92 | 76.92 |
| Particle size (nm) | Immediately after preparation | 142 | 237 | 118 |
| | After storage at 40°C for 1 week | 163 | 244 | 84 |
| Change in particle size (nm) after storage at 40°C for 1 week | | +21 | +7 | -34 |

As shown in Table 6, it can be found that the lipid nanoparticle of the present invention can solubilize various water-insoluble substances (MCT, CHE, or OA), and is excellent in emulsifying capacity (ability to generate smaller particles) and emulsion stability (ability to suppress particle size increase during storage).

[Test Example 7: Variation Evaluation of Encapsulated Substance]

<Production of Hyaluronic Acid Derivative>

The hyaluronic acid derivative was produced in the same manner as in Test Example 1.

<Production of Lipid Nanoparticle>

A lipid nanoparticle encapsulating a nucleic acid-lipid complex was prepared by the following method.
PC, CH, and oleylamine were dissolved in ethanol in an amount of 0.25 mass%, 0.5 mass%, and 1.25 mass%, respectively, to obtain an ethanol solution. While a solution in which 1 mg of salmon testis-derived deoxyribonucleic acid and 1.2 mg of the hyaluronic acid derivative had been dissolved in 9 mL of 100 mM acetate buffer (pH 1.5) was being vigorously stirred, 1 mL of the ethanol solution was added dropwise to prepare a lipid nanoparticle encapsulating a nucleic acid-lipid complex (Example 13).

<Evaluation of Particle Size and Nucleic Acid Encapsulation Rate>

The particle size and the nucleic acid encapsulation rate of the lipid nanoparticle of Example 13 was measured immediately after preparation. The particle size was measured by a particle diameter measuring device (ZETASIZER NANO, manufactured by Malvern Panalytical) using a dynamic light scattering method. The nucleic acid encapsulation rate was measured by the following method. The prepared lipid nanoparticle was passed through an ultrafiltration membrane (trade name: VIVASPIN 20, manufactured by Sartorius AG, molecular weight cutoff: 100 KDa), and the filtered outer aqueous phase was used as a measurement sample. The measurement sample was measured for the absorption wavelength at 260 nm using a spectrophotometer (manufactured by SHIMADZU CORPORATION), together with prepared 0.1 to 0.001 mg/mL DNA solutions for quantification, to quantify the DNA concentration. The nucleic acid encapsulation rate was calculated by the following equation. Here, the total DNA concentration is a concentration corresponding to the total amount of the used salmon testis-derived deoxyribonucleic acid, and is 0.1 mg/mL.

Nucleic acid encapsulation rate = (Total DNA concentration - Outer aqueous phase DNA concentration)/Total DNA concentration

The results are shown in Table 7.

[Table 7]

| | | Example 13 |
|---|---|---|
| PC (mass%) | | 0.025 |
| CH (mass%) | | 0.050 |
| Oleylamine (mass%) | | 0.125 |
| Salmon testis-derived deoxyribonucleic acid (mass%) | | 0.010 |
| Hyaluronic acid derivative (mass%) | | 0.012 |
| Ethanol (mass%) | | 10.000 |
| 100 mM acetate buffer (mass%) | | 89.778 |
| Ratio (PC : Encapsulated substance : Hyaluronic acid derivative) | | 1 :5.4 : 0.5 |
| Phospholipid content (mass%) | | 11.26 |
| Salt content (mass%) | | 0.00 |
| Encapsulated substance content (mass%) | | 60.81 |
| Hyaluronic acid derivative content (mass%) | | 5.41 |
| Particle size (nm) | Immediately after preparation | 163 |
| Nucleic acid encapsulation rate (%) | | 70.3 |

As shown in Table 7, it can be found that the lipid nanoparticle of the present invention can highly efficiently solubilize a nucleic acid-lipid complex (a complex of oleylamine as a cationic lipid and a nucleic acid) (nucleic acid encapsulation rate 70.3 %), and is excellent in emulsifying capacity (ability to generate smaller particles).

[Test Example 8: Variation Evaluation of Encapsulated Substance]

<Production of Hyaluronic Acid Derivative>

The hyaluronic acid derivative was produced in the same manner as in Test Example 1.

<Production of Lipid Nanoparticle>

A lipid nanoparticle encapsulating CsA was prepared by the following method.

PC and CsA were dissolved in ethanol in an amount of 10 mass% and 1 mass%, respectively, to obtain an ethanol solution. While a solution in which 250 mg of the hyaluronic acid derivative had been dissolved in 4.5 mL of PBS was being vigorously stirred, 0.5 mL of the ethanol solution was added dropwise to prepare a lipid nanoparticle encapsulating CsA (Example 14). Besides, a lipid nanoparticle encapsulating CsA was prepared in the same manner as in Example 14 except that no hyaluronic acid derivative was contained in PBS (Comparative Example 6).

<Evaluation of Particle Size and Appearance>

The particle size and appearance of each of the lipid nanoparticles of Example 14 and Comparative Example 6 was measured and observed immediately after preparation. The particle size was measured by a particle diameter measuring device (ZETASIZER NANO, manufactured by Malvern Panalytical) using a dynamic light scattering method. The appearance was visually observed. The results are shown in Table 8.

[Table 8]

| | Comparative Example 6 | Example 14 |
|---|---|---|
| PC (mass%) | 1.0 | 1.0 |
| CsA (mass%) | 0.1 | 0.1 |
| Hyaluronic acid derivative (mass%) | - | 5.0 |
| Ethanol (mass%) | 10.0 | 10.0 |

(continued)

| | | Comparative Example 6 | Example 14 |
|---|---|---|---|
| PBS (mass%) | | 88.9 | 83.9 |
| Ratio (PC : CsA : Hyaluronic acid derivative) | | 10 : 1 : 0 | 10 : 1 : 50 |
| Phospholipid content (mass%) | | 90.9 | 16.4 |
| Salt content (mass%) | | 72.7 | 12.4 |
| Encapsulated substance content (mass%) | | 9.1 | 1.6 |
| Hyaluronic acid derivative content (mass%) | | - | 82.0 |
| Particle size (nm) | Immediately after preparation | 603 | 447 |
| Appearance | | Precipitation observed White turbidity | No precipitation Slightly clear |

As shown in Table 8, it can be found that the lipid nanoparticle of the present invention can solubilize various water-insoluble substances (CsA, in the Test Example), and is excellent in emulsifying capacity (ability to generate smaller particles). In addition, it can be confirmed that the hyaluronic acid derivative can be blended without any problem even when contained in an amount of 5 mass%.

[Test Example 9: Variation Evaluation of Encapsulated Substance]

<Production of Hyaluronic Acid Derivative>

The hyaluronic acid derivative was produced in the same manner as in Test Example 1.

<Production of Lipid Nanoparticle>

A lipid nanoparticle encapsulating a water-insoluble substance (CsA or Tac) was prepared by the following method.

PC, a water-insoluble substance (CsA or Tac), and ethanol were mixed, and the mixture was heated to 60°C to form an oil phase. The hyaluronic acid derivative was dissolved in PBS to form an aqueous phase. The oil phase was added dropwise while the aqueous phase heated at 60°C was being stirred, and coarse emulsification was performed for 5 minutes. The coarse emulsion was treated 10 times at 150 MPa with a high pressure emulsifier (MICROFLUIDIZER, manufactured by MICROFLUIDICS) to prepare a lipid nanoparticle encapsulating a water-insoluble substance (CsA or Tac) (Examples 15 to 17). The encapsulated substance is water-insoluble and therefore is not present in the outer phase. The encapsulated substance present in the suspension is all solubilized in the lipid nanoparticle.

<Evaluation of Particle Size, Appearance, and Encapsulated Substance Content in Suspension>

The lipid nanoparticle of Examples 15 to 17 was measured immediately after preparation and after storage at 40°C for 1 week for its particle size, appearance, and encapsulated substance content of the water-insoluble substance (CsA or Tac) in the suspension. The particle size was measured by a particle diameter measuring device (ZETASIZER NANO, manufactured by Malvern Panalytical) using a dynamic light scattering method. The appearance was visually observed. The encapsulated substance content in the suspension was measured with high performance liquid chromatography (manufactured by Nihon Waters K.K.). Specifically, the column used was WATERS ECLIPSE XDB-C18 (4.6 × 150 mm, 5 μm), the mobile phase was acetonitrile : water : trifluoroacetic acid = 75 : 25 : 0.2, the detection method was UV (210 nm), the flow rate at the time of measuring CsA was 1.2 mL/min, the temperature was 60°C, the flow rate at the time of measuring Tac was 1.0 mL/min, and the temperature was 40°C, and the content of each of CsA and Tac in the suspension was quantified. The results are shown in Table 9.

[Table 9]

| | Example 15 | Example 16 | Example 17 |
|---|---|---|---|
| PC (mass%) | 2.0 | 2.0 | 4.0 |
| CsA (mass%) | 0.1 | - | 0.1 |

(continued)

| | | Example 15 | Example 16 | Example 17 |
|---|---|---|---|---|
| Tac (mass%) | | - | 0.1 | - |
| Hyaluronic acid derivative (mass%) | | 0.3 | 1.0 | 1.0 |
| Ethanol (mass%) | | 0.8 | 0.8 | 1.0 |
| PBS (mass%) | | 96.8 | 96.1 | 93.9 |
| Ratio (PC: Encapsulated substance : Hyaluronic acid derivative) | | 20 : 1 : 3 | 20 : 1 : 10 | 40 : 1 : 10 |
| Phospholipid content (mass%) | | 83.3 | 64.5 | 78.4 |
| Salt content (mass%) | | 36.6 | 28.1 | 16.7 |
| Encapsulated substance content (mass%) | | 4.2 | 3.2 | 2.0 |
| Hyaluronic acid derivative content (mass%) | | 12.5 | 32.3 | 19.6 |
| Particle size (nm) | Immediately after preparation | 34 | 75 | 48 |
| | After storage at 40°C for 1 week | 37 | 68 | 54 |
| Encapsulated substance content in suspension (mg/mL) | Immediately after preparation | 1 | 0.85 | 0.86 |
| | After storage at 40°C for 1 week | 0.95 | 0.71 | 0.83 |
| Appearance | Immediately after preparation | Clear | Clear | Slightly clear |
| | After storage at 40°C for 1 week | Clear | Clear | Slightly clear |

As shown in Table 9, it can be found that the lipid nanoparticle of the present invention can solubilize various water-insoluble substances (CsA or Tac), and is excellent in emulsifying capacity (ability to generate smaller particles) and emulsion stability (ability to suppress particle size increase during storage).

[Test Example 10: Variation Evaluation of Phospholipid and Encapsulated Substance]

<Production of Hyaluronic Acid Derivative>

The hyaluronic acid derivative was produced in the same manner as in Test Example 1.

<Production of Lipid Nanoparticle>

A lipid nanoparticle encapsulating a water-insoluble substance (LCT) was prepared by the following method.

PL and LCT were mixed and heated to 60°C to form an oil phase. The hyaluronic acid derivative was dissolved in PBS to form an aqueous phase. The oil phase was added dropwise while the aqueous phase heated at 60°C was being stirred, and coarse emulsification was performed for 5 minutes. The coarse emulsion was treated 5 times at 150 MPa with a high pressure emulsifier (MICROFLUIDIZER, manufactured by MICROFLUIDICS) to prepare a lipid nanoparticle encapsulating LCT (Examples 18 to 19). Besides, a lipid nanoparticle encapsulating LCT was prepared in the same manner as in Examples 18 and 19 except that no PL was contained in the oil phase (Comparative Example 7). Besides, a lipid nanoparticle encapsulating LCT was prepared in the same manner as in Examples 18 and 19 except that no hyaluronic acid derivative was contained in the aqueous phase (Comparative Example 8).

<Evaluation of Particle Size>

Each of the lipid nanoparticle suspensions prepared in Comparative Examples 7 to 8 and Examples 18 to 19 was weighed in a vial in an amount of 10 mL, added with 90 mg of sodium chloride, and then sealed and stored at 40°C. The particle size was measured immediately after preparation and after storage at 40°C for 1 week. The particle size was measured by a particle diameter measuring device (ZETASIZER NANO, manufactured by Malvern Panalytical) using a dynamic light scattering method. The results are shown in Table 10.

[Table 10]

| | | Comparative Example 7 | Comparative Example 8 | Example 18 | Example 19 |
|---|---|---|---|---|---|
| PL (mass%) | | - | 1.0 | 1.2 | 15.0 |
| LCT (mass%) | | 20.0 | 20.0 | 20.0 | 10.0 |
| Hyaluronic acid derivative (mass%) | | 1.0 | - | 1.0 | 1.0 |
| PBS (mass%) | | 79.0 | 79.0 | 77.8 | 74.0 |
| Ratio (PL : LCT : Hyaluronic acid derivative) | | 0 : 20 : 1 | 1 : 20 : 0 | 1 : 20 : 1 | 15 : 10 : 1 |
| Phospholipid content (mass%) | | - | 4.8 | 5.4 | 57.7 |
| Salt content (mass%) | | 3.4 | 3.4 | 3.2 | 2.6 |
| Encapsulated substance content (mass%) | | 95.2 | 95.2 | 90.1 | 38.5 |
| Hyaluronic acid derivative content (mass%) | | 4.8 | - | 4.5 | 3.8 |
| Particle size (nm) | Immediately after preparation | 212 | 193 | 183 | 106 |
| | After storage at 40°C for 1 week | Separation | Separation | 196 | 125 |

When a water-insoluble substance (LCT) is solubilized with a phospholipid alone (Comparative Example 8) or a hyaluronic acid derivative alone (Comparative Example 7), the emulsion stability is extremely low. On the other hand, when a phospholipid and a hyaluronic acid derivative are used in combination, significant improvement is observed in emulsion stability (ability to suppress particle size increase during storage) (Example 18 and Example 19).

[Test Example 11: Variation Evaluation of Encapsulated Substance]

<Production of Hyaluronic Acid Derivative>

The hyaluronic acid derivative was produced in the same manner as in Test Example 1.

<Production of Lipid Nanoparticle>

A lipid nanoparticle encapsulating water-insoluble substances (MCT and RD, or MCT and NP) was prepared by the following method.

PL, MCT, a drug that is a water-insoluble substance (RD or NP), and an organic solvent (ethanol or dimethylformamide (DMF)) were mixed, and the mixture was heated to 60°C to form an oil phase. The hyaluronic acid derivative was dissolved in pure water or a mixture of pure water and 1 mol/L aqueous sodium hydroxide solution to form an aqueous phase. The oil phase was added dropwise while the aqueous phase heated at 60°C was being stirred, and coarse emulsification was performed for 5 minutes. The coarse emulsion was treated 5 times at 150 MPa with a high pressure emulsifier (MICROFLUIDIZER, manufactured by MICROFLUIDICS) to prepare a lipid nanoparticle encapsulating water-insoluble substances (MCT and RD, or MCT and NP) (Examples 20 to 22).

<Evaluation of Particle Size>

The particle size of each of the lipid nanoparticles of Examples 20 to 22 was measured immediately after preparation. The particle size was measured by a particle diameter measuring device (ZETASIZER NANO, manufactured by Malvern Panalytical) using a dynamic light scattering method. The results are shown in Table 11. Here, the 1 mol/L aqueous sodium hydroxide solution contains 0.4 mass% of sodium hydroxide.

[Table 11]

| | | Example 20 | Example 21 | Example 22 |
|---|---|---|---|---|
| PL (mass%) | | 15.00 | 1.20 | 10.00 |
| MCT (mass%) | | 10.00 | 10.00 | 1.20 |
| RD (mass%) | | 2.00 | - | - |
| NP (mass%) | | - | 0.02 | 0.02 |
| Hyaluronic acid derivative (mass%) | | 1.00 | 1.00 | 1.00 |
| Ethanol (mass%) | | - | 0.80 | 0.80 |
| DMF (mass%) | | 5.00 | - | - |
| 1 mol/L aqueous sodium hydroxide solution (mass%) | | 10.00 | - | - |
| Pure water (mass%) | | 57.00 | 86.98 | 86.98 |
| Ratio (PC : Encapsulated substance : Hyaluronic acid derivative) | | 15 : 12 : 1 | 1.2 : 10.02 : 1 | 10 : 1.22 : 1 |
| Phospholipid content (mass%) | | 54.00 | 9.82 | 81.83 |
| Salt content (mass%) | | 0.14 | 0.00 | 0.00 |
| Encapsulated substance content (mass%) | | 42.86 | 82.00 | 9.98 |
| Hyaluronic acid derivative content (mass%) | | 3.60 | 8.18 | 8.18 |
| Particle size (nm) | Immediately after preparation | 204 | 119 | 58 |

As shown in Table 11, it can be found that the lipid nanoparticle of the present invention can solubilize various water-insoluble substances (MCT and RD, or MCT and NP), and is excellent in emulsifying capacity (ability to generate smaller particles).

[Test Example 12: Variation Evaluation of Hyaluronic Acid Derivative]

<Production of Hyaluronic Acid Derivative>

A hyaluronic acid derivative having a high modification rate was obtained in the same manner as in the hyaluronic acid derivative A of Test Example 5 except that the reaction time in a water bath at 80°C was prolonged to more than 8 hours. The modification rate of the obtained hyaluronic acid derivative was measured by $^{1}$H-NMR spectrum analysis.

<Production of Lipid Nanoparticle>

A lipid nanoparticle encapsulating LCT was prepared by the following method.

PL and LCT were mixed and heated to 60°C to form an oil phase. The hyaluronic acid derivative was dissolved (suspended) in PBS to form an aqueous phase. The oil phase was added dropwise while the aqueous phase heated at 60°C was being stirred, and coarse emulsification was performed for 5 minutes. The coarse emulsion was treated 5 times at 150 MPa with a high pressure emulsifier (MICROFLUIDIZER, manufactured by MICROFLUIDICS) to prepare a lipid nanoparticle encapsulating LCT (Example 23).

<Evaluation of Particle Size>

The particle size of the lipid nanoparticle of Example 23 was measured immediately after preparation and after storage at 40°C for 1 week. The particle size was measured by a particle diameter measuring device (ZETASIZER NANO, manufactured by Malvern Panalytical) using a dynamic light scattering method. The results are shown in Table 12.

[Table 12]

| | Example 23 |
|---|---|
| PL (mass%) | 1.0 |
| LCT (mass%) | 20.0 |

(continued)

| | | Example 23 |
|---|---|---|
| Hyaluronic acid derivative (mass%) | | 1.0 |
| PBS (mass%) | | 78.0 |
| Ratio (PL : LCT : Hyaluronic acid derivative) | | 1 : 20 : 1 |
| Average molecular weight of raw material hyaluronic acid | | 8000 |
| Number of carbon atoms in alkyl group represented by $R_3$ | | 12 to 13 |
| Modification rate of hyaluronic acid derivative (%) | | 47 |
| Phospholipid content (mass%) | | 4.5 |
| Salt content (mass%) | | 3.2 |
| Encapsulated substance content (mass%) | | 90.9 |
| Hyaluronic acid derivative content (mass%) | | 4.5 |
| Particle size (nm) | Immediately after preparation | 214 |
| | After storage at 40°C for 1 week | 218 |

As shown in Table 12, it can be found that the lipid nanoparticle of the present invention is excellent in emulsifying capacity (ability to generate smaller particles) and emulsion stability (ability to suppress particle size increase during storage) even when the modification rate of the hyaluronic acid derivative is changed.

## Claims

**1.** A production method for a lipid nanoparticle, the method comprising:

a step of dissolving a hyaluronic acid derivative in an aqueous solvent to form an aqueous phase;
a step of dissolving a phospholipid in an organic solvent to form an oil phase; and
a step of dropping and dispersing the oil phase into the aqueous phase,
wherein the hyaluronic acid derivative is represented by a following general formula (1):

[Chemical Formula 1]

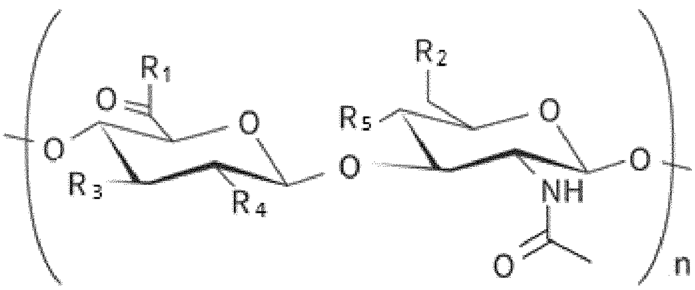

··· (1)

wherein, in the general formula (1), $R_1$, $R_2$, $R_3$, $R_4$, and $R_5$ each independently represent a hydroxy group or a group represented by $-O-CH_2-CH(OH)-CH_2-OR_6$; $R_6$ represents a linear or branched alkyl group or alkenyl group; and n represents an integer of 1 or more and 2500 or less, provided that a case where $R_1$, $R_2$, $R_3$, $R_4$, and $R_5$ are all hydroxy groups is excluded.

**2.** The production method according to claim 1, the method further comprising a step of subjecting a dispersion obtained in the dispersing step to a high-pressure emulsification treatment.

**3.** The production method according to claim 1 or 2, wherein the phospholipid is selected from the group consisting of

phosphatidylcholine, phosphatidylethanolamine, phosphatidylserine, phosphatidylglycerol, phosphatidylinositol, sphingomyelin, and a mixture of two or more thereof.

**4.** The production method according to any one of claims 1 to 3, wherein the $R_6$ is a linear alkyl group having 8 or more and 22 or less carbon atoms.

**5.** The production method according to any one of claims 1 to 4, wherein the hyaluronic acid derivative has a modification rate of 0.1% or more and 50% or less.

**6.** The production method according to any one of claims 1 to 5, wherein the lipid nanoparticle further includes a substance encapsulated in an outer shell made of at least the hyaluronic acid derivative and the phospholipid.

**7.** The production method according to claim 6, wherein the substance is a water-insoluble substance.

**8.** The production method according to claim 6 or 7, wherein the substance is selected from the group consisting of a water-insoluble drug, a complex of a nucleic acid and a cationic lipid, an oil, and a mixture of two or more thereof.

**9.** A lipid nanoparticle comprising:

a hyaluronic acid derivative; and
a phospholipid,
wherein the hyaluronic acid derivative is represented by a following general formula (1):

[Chemical Formula 2]

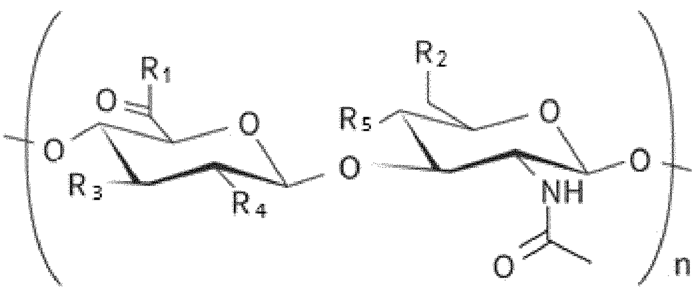

··· (1)

wherein, in the general formula (1), $R_1$, $R_2$, $R_3$, $R_4$, and $R_5$ each independently represent a hydroxy group or a group represented by $-O-CH_2-CH(OH)-CH_2-OR_6$; $R_6$ represents a linear or branched alkyl group or alkenyl group; and n represents an integer of 1 or more and 2500 or less, provided that a case where $R_1$, $R_2$, $R_3$, $R_4$, and $R_5$ are all hydroxy groups is excluded.

**10.** The lipid nanoparticle according to claim 9, wherein the phospholipid is selected from the group consisting of phosphatidylcholine, phosphatidylethanolamine, phosphatidylserine, phosphatidylglycerol, phosphatidylinositol, sphingomyelin, and a mixture of two or more thereof.

**11.** The lipid nanoparticle according to claim 9 or 10, wherein the $R_6$ is a linear alkyl group having 8 or more and 22 or less carbon atoms.

**12.** The lipid nanoparticle according to any one of claims 9 to 11, wherein the hyaluronic acid derivative has a modification rate of 0.1% or more and 50% or less.

**13.** The lipid nanoparticle according to any one of claims 9 to 12, further comprising a substance encapsulated in an outer shell made of at least the hyaluronic acid derivative and the phospholipid.

**14.** The lipid nanoparticle according to claim 13, wherein the substance is a water-insoluble substance.

**15.** The lipid nanoparticle according to claim 13 or 14, wherein the substance is selected from the group consisting of a

water-insoluble drug, a complex of a nucleic acid and a cationic lipid, an oil, and a mixture of two or more thereof.

**INTERNATIONAL SEARCH REPORT**

International application No. **PCT/JP2023/013650**

**A. CLASSIFICATION OF SUBJECT MATTER**

***A61K 48/00***(2006.01)i; ***A61P 31/12***(2006.01)i; ***A61K 9/10***(2006.01)i; ***A61K 9/51***(2006.01)i; ***A61K 47/24***(2006.01)i; ***A61K 47/36***(2006.01)i; ***A61K 31/7088***(2006.01)i
FI: A61K47/36; A61K47/24; A61K9/51; A61K9/10; A61K48/00; A61K31/7088; A61P31/12

According to International Patent Classification (IPC) or to both national classification and IPC

**B. FIELDS SEARCHED**

Minimum documentation searched (classification system followed by classification symbols)

A61K48/00; A61P31/12; A61K9/10; A61K9/51; A61K47/24; A61K47/36; A61K31/7088

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2016-204341 A (IWASE COSFA K.K.) 08 December 2016 (2016-12-08) claims 1, 4, 5, paragraphs [0017], [0028], [0033], [0034], [0042], [0052], table 1, example 12 | 9-15<br>1-8 |
| A | WO 2011/102462 A1 (KEWPIE CORP.) 25 August 2011 (2011-08-25) entire text | 1-15 |
| A | US 2016/0151283 A1 (ICNODERM S. R. L.) 02 June 2016 (2016-06-02) entire text | 1-15 |
| A | JP 2019-189574 A (HOKKAIDO UNIVERSITY) 31 October 2019 (2019-10-31) entire text | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2023** | **23 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/013650**

| Patent document cited in search report | Publication date (day/month/year) | Patent family member(s) | Publication date (day/month/year) |
|---|---|---|---|
| JP 2016-204341 A | 08 December 2016 | (Family: none) | |
| WO 2011/102462 A1 | 25 August 2011 | US 2012/0316329 A1<br>entire text<br>JP 2012-21166 A<br>EP 2537867 A1<br>CN 102770459 A<br>KR 10-2012-0140242 A | |
| US 2016/0151283 A1 | 02 June 2016 | EP 3025732 A1 | |
| JP 2019-189574 A | 31 October 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019189574 A
- JP 2020530778 A
- JP 4845071 B


### Non-patent literature cited in the description


- **T. C. LAURENT** ; **M. RYAN** ; **A. PIETRUSZKIEWICZ**. *B.B.A.*, 1960, vol. 42, 476-485
- The Japanese Pharmacopoeia